# EUROPEAN PATENT APPLICATION

(11) **EP 3 884 993 A1**
(43) Date of publication of application: **29.09.2021**
(21) Application number: 18940599.6
(22) Date of filing: 23.11.2018
(51) Int. Cl.: A61N 1/05, A61N 1/362, A61B 17/34, A61B 17/00

(54) **DEVICE FOR VALVE REGURGITATION SURGERY AND CARDIAC PACEMAKER LEAD FIXATION**

(71) Applicant: Taupnu Medical Co., Ltd., Busan 46241 (KR)
(72) Inventor: KIM, June Hong, Busan 48516 (KR)
(74) Representative: dompatent von Kreisler Selting Werner - Partnerschaft von Patent- und Rechtsanwälten mbB
(86) International application number: PCT/KR2018/014554
(87) International publication number: WO 2020/105768

(57) **Abstract**

The present disclosure relates to a device for valve regurgitation surgery and pacemaker lead fixation, and more particularly, to a device for valve regurgitation surgery and pacemaker lead fixation that allows mitral regurgitation surgery, tricuspid regurgitation surgery, and fixation of a pacemaker lead to an interventricular septum to be performed using a single device. The device for valve regurgitation surgery and pacemaker lead fixation according to the present disclosure includes a cerclage wire, a coronary sinus tube which has a lumen formed to insert the cerclage wire thereinto and which is inserted into a coronary sinus, a tricuspid valve tube which has a lumen formed to insert the cerclage wire thereinto and which crosses a tricuspid valve and protects tissues of the tricuspid valve and the interventricular septum, a lead insertion tube which is coupled to the tricuspid valve tube and has an end facing the interventricular septum, and a pacemaker lead which is inserted into the lead insertion tube and has an end inserted into the bundle of His.

## Description

### [Technical Field]

The present disclosure relates to a device for valve regurgitation surgery and pacemaker lead fixation, and more particularly, to a device for valve regurgitation surgery and pacemaker lead fixation that allows mitral regurgitation surgery, tricuspid regurgitation surgery, and fixation of a pacemaker lead to an interventricular septum to be performed using a single device.

### [Background Art]

The heart consists of two atriums and two ventricles and is connected to four types of blood vessels including the aorta, vena cava, pulmonary artery, and pulmonary vein to form a passage of blood flow.

Based on the interventricular septum located at the center of the heart, one side is divided into the right atrium and right ventricle, and the other side is divided into the left atrium and left ventricle. The tricuspid valve is present between the right atrium and right ventricle, and the mitral valve is present between the left atrium and left ventricle.

The heart repeatedly contracts and relaxes and serves as a pump such that blood flows along blood vessels. During systole of the heart, blood in the heart flows toward blood vessels. In the right side of the heart, blood flows from the right ventricle to the pulmonary artery, and in the left side of the heart, blood flows from the left ventricle to the aorta.

However, when a valve between an atrium and a ventricle does not function well, blood in the ventricle flows backwards during systole of the heart. When the tricuspid valve between the right atrium and right ventricle does not function properly and blood in the right ventricle flows backwards to the right atrium, it is referred to as "tricuspid regurgitation," and when the mitral valve between the left atrium and left ventricle does not function properly and blood in the left ventricle flows backwards to the left atrium, it is referred to as "mitral regurgitation."

Tricuspid regurgitation often occurs in patients with mitral regurgitation. In patients with heart valve disease, as heart function declines, atrial fibrillation, arrhythmia, heart failure and the like may occur, and a pacemaker may be implanted in the body to treat such conditions.

The onsets of the tricuspid regurgitation, mitral regurgitation, and heart failure are linked to each other, and some patients have the three diseases at the same time. To this end, there is a need for a device for treating the three diseases at one time.

The inventor of the present disclosure has filed a patent application or been granted a patent for a device for treating mitral regurgitation in Korean Patent Registration No. 10-1116867, Korean Patent Registration No. 10-1231140, Korean Patent Registration No. 10-1563172, Korean Patent Registration No. 10-1581021, and Korean Unexamined Patent Application Publication No. 10-2013-0074823, has filed a patent application for a device for treating tricuspid regurgitation in Korean Unexamined Patent Application Publication No. 10-2017-0044065 and Korean Unexamined Patent Application Publication No. 10-2015-0144568, and has filed a patent application for a device for placing an end of a pacemaker lead in Korean Unexamined Patent Application Publication No. 10-2016-0011530 and Korean Unexamined Patent Application Publication No. 10-2016-0020887.

The present disclosure is part of ongoing research and is for allowing mitral regurgitation surgery, tricuspid regurgitation surgery, and placement of an end of a pacemaker lead in an interventricular septum to be performed using a single device for a patient who needs all the three procedures.

### [Disclosure]

### [Technical Problem]

The present disclosure is for allowing treatment for mitral regurgitation, treatment for tricuspid regurgitation, and fixation of a pacemaker lead to be performed using a single device.

The present disclosure is also directed to providing a device for valve regurgitation surgery and pacemaker lead fixation that uses a catheter, thus allowing minimally invasive surgery using a catheter technique, rather than a surgical method in which the chest of the human body is cut open to make an incision in the heart, to be easily performed and allowing the effect of surgery to be enhanced.

The objectives of the present disclosure are not limited to those mentioned above, and other unmentioned objectives should be clearly understood by those of ordinary skill in the art from the description below.

### [Technical Solution]

The present disclosure provides a device for valve regurgitation surgery and pacemaker lead fixation, the device including a cerclage wire, a coronary sinus tube which has a lumen formed to insert the cerclage wire thereinto and which is inserted into a coronary sinus, a tricuspid valve tube which has a lumen formed therein and which crosses a tricuspid valve and protects tissues of the tricuspid valve and an interventricular septum, and a pacemaker lead which has an end inserted into the bundle of His.

An arch portion configured to protect the coronary sinus may be coupled to one side of the cerclage wire.

An upper portion of the pacemaker lead may be coupled to the tricuspid valve tube, and a lower portion of the pacemaker lead may be separated therefrom.

The end of the pacemaker lead may be formed in the shape of a screw to easily penetrate the interventricular septum.

A direction control device may be installed at one side of the pacemaker lead, and the other side of the pacemaker lead may be bent or straightened as the direction control device is controlled.

According to another embodiment, a device for valve regurgitation surgery and pacemaker lead fixation includes a cerclage wire, a coronary sinus tube which has a lumen formed to insert the cerclage wire thereinto and which is inserted into a coronary sinus, a tricuspid valve tube which has a lumen formed to insert the cerclage wire thereinto and which crosses a tricuspid valve and protects tissues of the tricuspid valve and an interventricular septum, a lead insertion tube which is coupled to the tricuspid valve tube and has an end facing the interventricular septum, and a pacemaker lead which is inserted into the lead insertion tube and has an end inserted into the bundle of His.

An arch portion configured to protect the coronary sinus may be coupled to one side of the cerclage wire.

A direction control device may be installed at one side of the pacemaker lead, and the other side of the pacemaker lead may be bent or straightened as the direction control device is controlled.

The end of the pacemaker lead may be formed in the shape of a screw to easily penetrate the interventricular septum.

### [Advantageous Effects]

A device for valve regurgitation surgery and pacemaker lead fixation according to the present disclosure allows treatment for mitral regurgitation, treatment for tricuspid regurgitation, and fixation of a pacemaker lead for treatment for heart failure to be performed using a single device for patients who need at least one of the above procedures.

Also, since an end of the pacemaker lead detects an electrocardiogram and is inserted into the bundle of His, electrical stimulation can be delivered more efficiently.

### [Description of Drawings]

FIG. 1 is a perspective view of a device for valve regurgitation surgery and pacemaker lead fixation according to an exemplary embodiment of the present disclosure.
FIG. 2 is a perspective view of a pacemaker lead according to an exemplary embodiment of the present disclosure.
FIG. 3 is a perspective view illustrating another example of the device for valve regurgitation surgery and pacemaker lead fixation according to an exemplary embodiment of the present disclosure that is illustrated in FIG. 1.
FIG. 4 is a perspective view of a device for valve regurgitation surgery and pacemaker lead fixation according to another exemplary embodiment of the present disclosure.
FIG. 5 is a perspective view illustrating another example of the device for valve regurgitation surgery and pacemaker lead fixation according to another exemplary embodiment of the present disclosure that is illustrated in FIG. 4.
FIG. 6 is a flowchart of a method of performing surgery using the device for valve regurgitation surgery and pacemaker lead fixation according to an exemplary embodiment of the present disclosure.
FIG. 7 is a picture of a capture catheter according to an exemplary embodiment of the present disclosure.

### <Description of reference numerals>

- 10:: cerclage wire
- 12:: arch portion
- 20:: coronary sinus tube
- 30:: tricuspid valve tube
- 32:: blocking portion
- 40:: lead insertion tube
- 50:: pacemaker lead
- 52:: body portion
- 54:: direction control device
- 60:: stopper
- 70:: capture catheter
- 72:: mesh

### [Modes of the Invention]

The advantages, features, and methods of achieving the same will become more apparent by referring to embodiments described in detail below with reference to the accompanying drawings. However, the present disclosure is not limited to the embodiments disclosed below and may be implemented in various different forms. The present embodiments make the disclosure of the present disclosure complete and are provided to completely inform those of ordinary skill in the art to which the present disclosure pertains of the scope of the present disclosure. The present disclosure is only defined by the scope of the claims.

Details for embodying the present disclosure will be described in detail with reference to the accompanying drawings. The same reference numerals refer to the same elements regardless of the drawings, and the term "and/or" may refer to each mentioned item or any combination of one or more of the mentioned items.

The terms used herein are for describing the embodiments and are not intended to limit the present disclosure. In the present specification, a singular expression includes a plural expression unless the context clearly indicates otherwise. The terms "comprises" and/or "comprising" used herein do not preclude the presence or addition of one or more elements other than those mentioned.

Unless defined otherwise, all terms including technical or scientific terms used herein may have the same meaning as commonly understood by those of ordinary skill in the art to which the present disclosure pertains. Also, terms defined in commonly used dictionaries should not be construed in an idealized or overly formal sense unless expressly so defined herein.

Hereinafter, exemplary embodiments of the present disclosure will be described in detail with reference to the accompanying drawings.

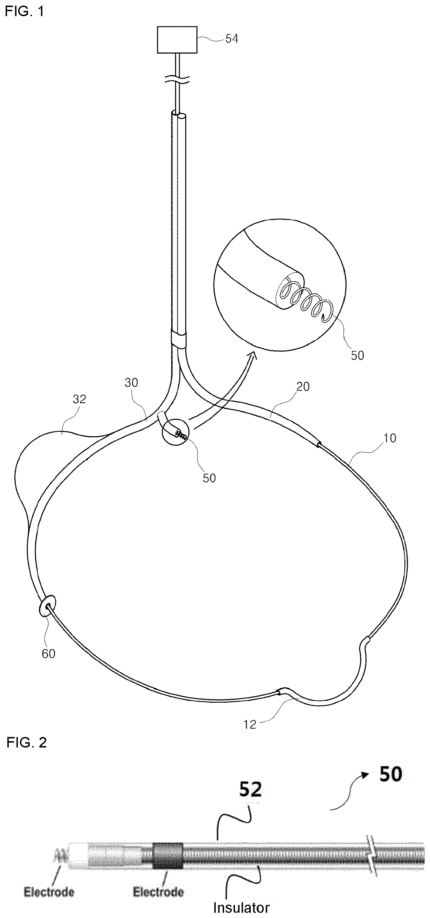

FIG. 1 is a perspective view of a device for valve regurgitation surgery and pacemaker lead fixation according to an exemplary embodiment of the present disclosure. Referring to FIG. 1, the device for valve regurgitation surgery and pacemaker lead fixation according to an exemplary embodiment of the present disclosure includes a cerclage wire 10, a coronary sinus tube 20, a tricuspid valve tube 30, and a pacemaker lead 50.

The cerclage wire 10 is a name given in the sense that it is connected to the coronary sinus, interventricular septum, and tricuspid valve in a circle. As illustrated in the drawing, the cerclage wire 10 is connected to the coronary sinus, interventricular septum, and tricuspid valve in one circle. When the cerclage wire 10 is pushed or pulled from above, the cerclage wire 10 maintains proper tension along the size and shape of the heart of a patient and grasps a mitral annulus to tighten the mitral annulus. Therefore, mitral regurgitation may be treated.

An arch portion 12 is formed at one side of the cerclage wire 10. The cerclage wire 10 is inserted into a coronary sinus, and when a coronary artery is located below the coronary sinus, considerable external pressure is applied to the coronary artery by the cerclage wire 10. The arch portion 12 prevents this and protects the coronary artery.

The coronary sinus tube 20 has a lumen formed to insert the cerclage wire 10 thereinto, and a lower portion of the coronary sinus tube 20 is inserted into the coronary sinus to protect the coronary sinus.

The tricuspid valve tube 30 has a lumen formed therein and passes through an orifice, which is formed due to incomplete closing of the tricuspid valve, to reach the interventricular septum. The tricuspid valve tube 30 includes a blocking portion 32. The blocking portion 32 is a portion configured to block the orifice formed due to the incomplete closing of the tricuspid valve, and a blocking membrane, a blocking balloon, or the like is used as the blocking portion 32. Due to the blocking portion 32, tricuspid regurgitation may be treated.

In order to prevent an end of the tricuspid valve tube 30 from penetrating into the interventricular septum, a stopper 60 is installed at a lower end of the tricuspid valve tube 30. This allows the tricuspid valve tube 30 to float instead of coming in close contact with a portion around the tricuspid valve to prevent damage to the tricuspid valve due to the tricuspid valve tube 30.

FIG. 2 is a perspective view of a pacemaker lead according to an exemplary embodiment of the present disclosure.

Referring to FIG. 2, an end of the pacemaker lead 50 is formed in the shape of a screw to easily penetrate the interventricular septum, and an outer portion of a body portion 52 of the pacemaker lead 50 is made of an insulator. Of course, the body portion 52 may not include an insulator. In the case of the pacemaker lead 50 that does not include an insulator, the body portion 52 is formed in a small thickness. Due to being able to detect an electrical signal of the heart, the pacemaker lead 50 may detect the location of the bundle of His and delivers electrical stimulation to the bundle of His or interventricular septum.

The pacemaker lead 50 is inserted into the tricuspid valve tube 30, and a lower portion of the pacemaker lead 50 is located outside the tricuspid valve tube 30. A direction control device 54 is installed at an upper end of the pacemaker lead 50, and the lower portion of the pacemaker lead 50 is bent or straightened as the direction control device 54 is controlled. Therefore, the direction control device 54 may be controlled to move the lower portion of the pacemaker lead 50 and detect an electrocardiogram of the interventricular septum to identify the location of the bundle of His, and the end of the pacemaker lead 50 is fixed to the identified location of the bundle of His.

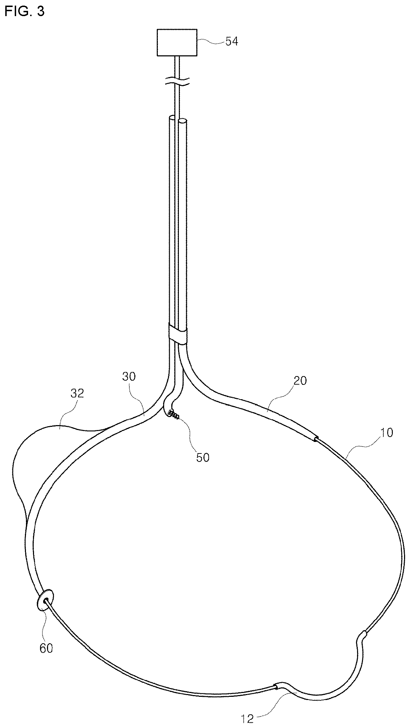

FIG. 3 is a perspective view illustrating another example of the device for valve regurgitation surgery and pacemaker lead fixation according to an exemplary embodiment of the present disclosure that is illustrated in FIG. 1.

Referring to FIG. 3, the pacemaker lead 50 may be coupled to an outer portion of the tricuspid valve tube 30. An upper portion of the pacemaker lead 50 is coupled to the tricuspid valve tube 30, and a lower portion of the pacemaker lead 50 is separated therefrom. Therefore, as the direction control device 54 coupled to the upper portion of the pacemaker lead 50 is controlled, the lower portion of the pacemaker lead 50 may be bent or straightened, and the pacemaker lead 50 may detect the location of the bundle of His.

Elements other than the pacemaker lead 50 are the same as those described above with reference to FIGS. 1 and 2.

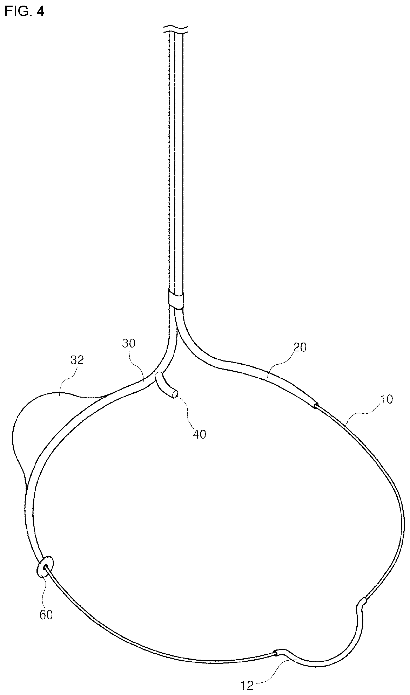

FIG. 4 is a perspective view of a device for valve regurgitation surgery and pacemaker lead fixation according to another exemplary embodiment of the present disclosure.

Referring to FIG. 4, the device for valve regurgitation surgery and pacemaker lead fixation according to an exemplary embodiment of the present disclosure includes the cerclage wire 10, the coronary sinus tube 20, the tricuspid valve tube 30, a lead insertion tube 40, and the pacemaker lead 50.

The lead insertion tube 40 has a lumen formed to insert the pacemaker lead 50 thereinto. An upper portion of the lead insertion tube 40 is inserted into and coupled to the tricuspid valve tube 30, a lower portion of the lead insertion tube 40 is separated from the tricuspid valve tube 30 and located outside the tricuspid valve tube 30, and a lower end of the lead insertion tube 40 is formed to face the interventricular septum.

The pacemaker lead 50 is inserted into the lead insertion tube 40, and when an end of the pacemaker lead 50 reaches the interventricular septum, the direction control device 54 is controlled to move the pacemaker lead 50 to detect an electrocardiogram of the interventricular septum. After the location of the bundle of His is detected, the end of the pacemaker lead 50 is fixed to the bundle of His.

Elements other than the lead insertion tube 40 are the same as those described above with reference to FIG. 1.

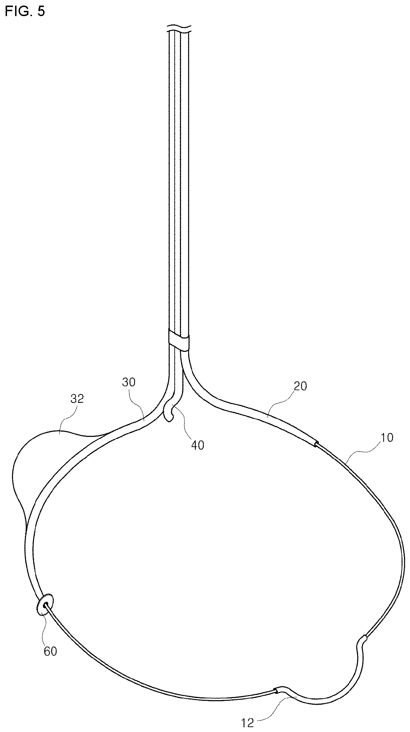

FIG. 5 is a perspective view illustrating another example of the device for valve regurgitation surgery and pacemaker lead fixation according to another exemplary embodiment of the present disclosure that is illustrated in FIG. 4.

Referring to FIG. 5, the lead insertion tube 40 may be coupled to the outer portion of the tricuspid valve tube 30, the upper portion of the lead insertion tube 40 is coupled to the upper portion of the tricuspid valve tube 30 while the lower portion of the lead insertion tube 40 is separated therefrom, and an end of the lead insertion tube 40 faces the interventricular septum.

The pacemaker lead 50 is inserted into the lead insertion tube 40, and when the end of the pacemaker lead 50 reaches the interventricular septum, the direction control device 54 is controlled to move the pacemaker lead 50 to detect the electrocardiogram of the interventricular septum. After the location of the bundle of His is detected, the end of the pacemaker lead 50 is fixed to the bundle of His.

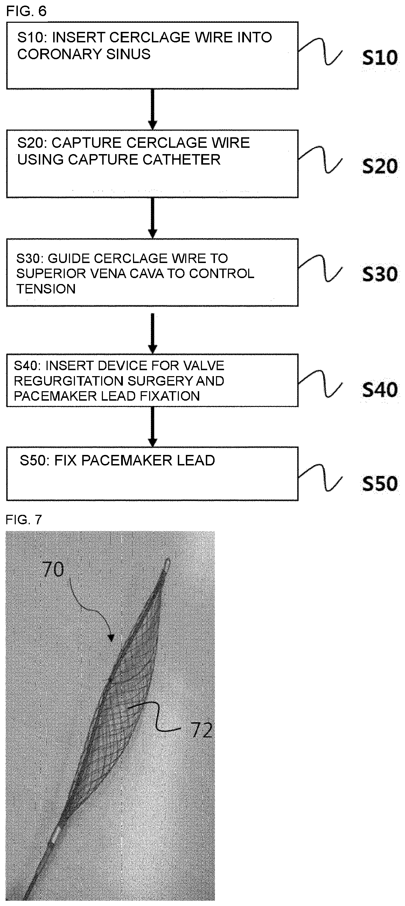

FIG. 6 is a flowchart of a method of performing surgery using the device for valve regurgitation surgery and pacemaker lead fixation according to an exemplary embodiment of the present disclosure.

Referring to FIG. 6, first, inserting a cerclage wire into a coronary sinus (S10) is a step in which the cerclage wire 10 is moved to the superior vena cava, coronary sinus, and septal vein.

A balloon catheter (not illustrated) may be included to facilitate movement of the cerclage wire 10. The balloon catheter (not illustrated) blocks blood flow in the coronary sinus and causes swelling of the coronary sinus to make it easy to view the septal vein, which is generally difficult to identify.

The cerclage wire 10 passes through the interventricular septum through the septal vein and moves to the right ventricle. The cerclage wire 10 is formed to have a sharp end to pass through the interventricular septum, but when it is difficult to penetrate the interventricular septum just with only the cerclage wire 10, a puncture catheter (not illustrated) is necessary. The puncture catheter (not illustrated) serves to support the cerclage wire 10 when the cerclage wire 10 punctures the interventricular septum and allows the cerclage wire 10 to more easily pass through the interventricular septum.

Next, a capture catheter is used to capture the cerclage wire (S20). As illustrated in FIG. 7, a capture catheter 70 includes a mesh 72, and the mesh 72 may be narrowed or widened by a force applied to the capture catheter 70 from outside the body. The capture catheter 70 moves to the inferior vena cava, tricuspid valve, and right ventricle, and a safe zone passage catheter (not illustrated) may be further included to allow the capture catheter 70 to safely move inside the body. The mesh 72 is narrowed while moving to the right ventricle and is widened in the right ventricle, and the cerclage wire 10 is inserted into the mesh 72. The mesh 72 is narrowed again, and after the cerclage wire 10 is captured, the mesh 72 exits the body through the inferior vena cava. Here, one end of the cerclage wire 10 is located at the superior vena cava, and the other end of the cerclage wire 10 is located at the inferior vena cava.

Next, the cerclage wire is guided to the superior vena cava to control tension (S30). The cerclage wire 10 guided to the inferior vena cava is guided to the superior vena cava again by a snare or forceps inserted into the superior vena cava, and both ends of the cerclage wire 10 are located outside the body through the superior vena cava. Therefore, the cerclage wire 10 passes through the superior vena cava, coronary sinus, septal vein, interventricular septum, and right ventricle and is located at the superior vena cava again, and in this way, the cerclage wire 10 is in a circular shape, and both ends thereof are located outside the body. The cerclage wire 10 is pushed or pulled to maintain proper tension along the size and shape of the heart of a patient, grasp and tighten the mitral annulus, and completely close the mitral valve. Thus, mitral regurgitation may be treated.

Next, the device for valve regurgitation surgery and pacemaker lead fixation is inserted (S40). Both ends of the cerclage wire 10 located outside the body are inserted into the coronary sinus tube 20 and the tricuspid valve tube 30, respectively, and then the coronary sinus tube 20 and the tricuspid valve tube 30 are inserted into the body along the cerclage wire 10. Here, preferably, the pacemaker lead 50 coupled to the tricuspid valve tube 30 is inserted toward the right atrium together with the tricuspid valve tube 30.

The stopper 60 is coupled to the lower end of the tricuspid valve tube 30. Alternatively, the stopper 60 may be coupled to a lower portion of the tricuspid valve tube 30. This allows the tricuspid valve tube 30 to float instead of coming in close contact with a portion around the tricuspid valve to prevent damage to the tricuspid valve. Also, the tricuspid valve tube 30 includes the blocking portion 32. The blocking portion 32 passes through an orifice formed due to the incomplete closing of the tricuspid valve so that the tricuspid valve is completely closed. The blocking portion 32 may be in the form of a blocking membrane, a blocking balloon, or the like.

Lastly, the pacemaker lead is fixed (S50). The direction control device 54 may be installed at the upper end of the pacemaker lead 50 and may be controlled so that the lower portion of the pacemaker lead 50 is bent or straightened. Using the direction control device 54, the lower portion of the pacemaker lead 50 is moved to detect an electrocardiogram of the interventricular septum to identify the location of the bundle of His, and the end of the pacemaker lead 50 is fixed to the identified location of the bundle of His.

According to the steps described above, mitral regurgitation surgery, tricuspid regurgitation surgery, and fixation of a pacemaker lead may be performed using a single device. Not only is it possible to simultaneously perform the three procedures using a single device, but it is also possible to use the device for at least one or more of the three purposes (treatment for mitral regurgitation, treatment for tricuspid regurgitation, and fixation of a pacemaker lead for treatment for heart failure).

Also, since an end of the pacemaker lead detects an electrocardiogram and is inserted into the bundle of His, electrical stimulation may be delivered more efficiently.

The embodiments of the present disclosure have been described above with reference to the accompanying drawings, but those of ordinary skill in the art to which the present disclosure pertains should understand that the present disclosure may be embodied in other specific forms without changing the technical idea or essential features of the present disclosure. Therefore, the embodiments described above should be understood as being illustrative, instead of limiting, in all aspects.

## Claims

1. A device for valve regurgitation surgery and pacemaker lead fixation, the device comprising:
a cerclage wire;
a coronary sinus tube which has a lumen formed to insert the cerclage wire thereinto and which is inserted into a coronary sinus;
a tricuspid valve tube which has a lumen formed therein and which crosses a tricuspid valve and protects tissues of the tricuspid valve and an interventricular septum; and
a pacemaker lead which has an end inserted into the bundle of His.

2. The device of claim 1, wherein an arch portion configured to protect the coronary sinus is coupled to one side of the cerclage wire.

3. The device of claim 1, wherein an upper portion of the pacemaker lead is coupled to the tricuspid valve tube, and a lower portion of the pacemaker lead is separated therefrom.

4. The device of claim 1, wherein the end of the pacemaker lead is formed in the shape of a screw to easily penetrate the interventricular septum.

5. The device of claim 1, wherein a direction control device is installed at one side of the pacemaker lead, and the other side of the pacemaker lead is bent or straightened as the direction control device is controlled.

6. A device for valve regurgitation surgery and pacemaker lead fixation, the device comprising:
a cerclage wire;
a coronary sinus tube which has a lumen formed to insert the cerclage wire thereinto and which is inserted into a coronary sinus;
a tricuspid valve tube which has a lumen formed to insert the cerclage wire thereinto and which crosses a tricuspid valve and protects tissues of the tricuspid valve and an interventricular septum;
a lead insertion tube which is coupled to the tricuspid valve tube and has an end facing the interventricular septum; and
a pacemaker lead which is inserted into the lead insertion tube and has an end inserted into the bundle of His.

7. The device of claim 6, wherein an arch portion configured to protect the coronary sinus is coupled to one side of the cerclage wire.

8. The device of claim 6, wherein a direction control device is installed at one side of the pacemaker lead, and the other side of the pacemaker lead is bent or straightened as the direction control device is controlled.

9. The device of claim 6, wherein the end of the pacemaker lead is formed in the shape of a screw to easily penetrate the interventricular septum.
